# EUROPÄISCHE PATENTSCHRIFT

(11) **EP 1 033 982 B1**
(45) Veröffentlichungstag und Bekanntmachung des Hinweises auf die Patenterteilung: **13.04.2005**
(21) Anmeldenummer: 98962368.1
(22) Anmeldetag: 20.11.1998
(51) Int. Cl.: A61K 31/135, A61K 31/27, A61P 33/02

(54) **MITTEL ZUR BEKÄMPFUNG VON NEOSPORA SPEC, DIE WIRKSTOFFE, DIE DAS MIKROTUBULINSYTEM VON PLANZEN HEMMEN, ENTHALTEN**
AGENTS FOR COMBATING NEOSPORA SPEC
AGENTS POUR LUTTER CONTRE NEOSPORA SPEC

(30) Priorität: 03.12.1997 DE 19753504
(43) Veröffentlichungstag der Anmeldung: 13.09.2000
(73) Patentinhaber: Bayer HealthCare AG, 51368 Leverkusen (DE)
(72) Erfinder: GREIF, Gisela, D-53424 Remagen (DE); LIEB, Folker, D-51375 Leverkusen (DE); FEDTKE, Carl, D-51069 Köln (DE)
(86) Internationale Anmeldenummer: PCT/EP1998/007460
(87) Internationale Veröffentlichungsnummer: WO 1999/027923

(56) Entgegenhaltungen:
- CHAN M.M. ET AL: "Plant microtubule inhibitors against trypanosomatids" PARASITOLOGY TODAY, Bd. 10, Nr. 11, 1994, Seiten 448-451, XP002101366
- DATABASE MEDLINE US NATIONAL LIBRARY OF MEDICINE (NLM), BETHESDA, MD, US STOKKERMANS T J ET AL: "Inhibition of Toxoplasma gondii replication by dinitroaniline herbicides." XP002101368 & EXPERIMENTAL PARASITOLOGY, (1996 DEC) 84 (3) 355-70. JOURNAL CODE: EQP. ISSN: 0014-4894., United States
- LINDSAY D.S. ET AL: "Examination of the activities of 43 chemotherapeutic agents against Neospora caninum tachyzoites in cultured cells" AMERICAN JOURNAL OF VETERINARY RESEARCH, Bd. 55, Nr. 7, 1994, Seiten 976-981, XP002101367

## Beschreibung

Die vorliegende Erfindung betrifft die Verwendung von Wirkstoffen, die das Mikrotubulinsystem von Pflanzen hemmen, zur Herstellung von Arzneimittel zur Bekämpfung von durch Protozoen der Gattung Neospora hervorgerufenen Erkrankungen.

Neosporose ist eine Erkrankung von Tieren hervorgerufen durch Parasiten der Gattung Neospora, insbesondere Neospora caninum. Neospora-Infektionen sind bekannt bei Hund, Rind, Schaf, Ziege, Pferd.

Der Endwirt von Neospora spec wie N. caninum und damit der vollständige Entwicklungszyklus des Parasiten sind noch nicht bekannt. Von dem Entwicklungszyklus dieses Parasiten sind bis jetzt nur die ungeschlechtlichen Vermehrungsstadien in Form der Schizogonie und der einzelligen Tachyzoiten/Bradyzoiten bekannt. Tachyzoiten sind einzellige infektiöse Parasitenstadien in der Größe 3-7 x 1-5 µm, die nach einer intrazellulären Vermehrung in Gewebecysten gebildet werden. Der Vorgang dieser Vermehrung heißt Endodyogenie.

Die Vermehrung über Tachyzoiten erfolgt bevorzugt in Organellen wie Muskeln und Nervenzellen. Daher treten die pathologischen Symptome nach einer natürlichen Infektion bevorzugt in diesen Geweben auf. So kommt es beim Hund nach einer natürlichen Infektion ab der 5. bis 6. Lebenswoche zu Krankheitssymptomen mit Anzeichen von Überempfindlichkeiten aufgrund von Nervenwurzelentzündungen und zunehmender Parese der Hinterbeine. Weitere histopathologische Befunde treten im Nervensystem, und zwar bevorzugt im Gehirn und Rückenmark auf. Hier dominieren ausgedehnte nichteitrige Entzündungen, Glia-Wucherungen und perivaskuläre Infiltrationen mit mononukleären Zellen (Makrophagen, Lymphocyten, wenige Plasmazellen) z.T. auch Eosinophile und Neutrophile. In der Muskulatur treten auch schon makroskopisch sichtbar nekrotisch-degenerative Veränderungen auf. Auffallend sind neben einer mehr oder weniger ausgeprägten Atrophie lange blasse Längsstreifen.

In Kalifornien und Australien gelten Neospora caninum Infektionen als Hauptursache für Aborte in Rinderherden.

Die Krankheitssymptome beim Rind ähneln denjenigen beim Hund. Es treten Ataxien auf, die Gelenkreflexe sind stark abgeschwächt und es treten Paresen an den Hinterbeinen, z.T. an allen vier Beinen auf. Die histologischen Bilder ähneln denen, wie sie bei Hunden zu finden sind: Im Vordergrund stehen nichteitrige Meningitis und Myelitis.

Für die in vivo-Wirksamkeit von Substanzen die zur Bekämpfung von Neosporose geeignet sind liegen bis jetzt nur sehr wenige Angaben vor, da adäquate in vivo-Testsysteme noch entwickelt werden müssen. Bei experimentell infizierten Mäusen erwies sich Sulfadiazin (über das Trinkwasser verabreicht) nur als wirksam, wenn die Behandlung prophylaktisch, d.h. vor der Infektion begann. Beim Hund hat die Behandlung mit Sulfadiazin und Clindamycin nur dann eine Erfolgschance, wenn sie sehr frühzeitig bei dem e rsten A uftreten k linischer S ymptome i nfolge d er N ervenwurzelentzündungen beginnt.

Die vorliegende Erfindung betrifft die Verwendung von Wirkstoffen, die das Mikrotubulinsystem von P flanzen h emmen, z ur H erstellung von Arzneimitteln z ur Bekämpfung von durch Protozoen der Gattung Neospora hervorgerufenen Erkrankungen.

Mikrotubuli (MT) gehören zu einem wichtigen Filament-Typ des Cytoskeletts. Hauptbestandteil dieser Struktur ist das Tubulin, ein globuläres Polypeptid mit einer molaren Masse von 50.000 Dalton. Bei der intrazellulären Aggregation der Mikrotubuli lagern sich Untereinheiten (Heterodimere) bestehend aus a- und β-Tubulin zunächst zu Protofilamenten zusammen, die dann eine Mikrotubulin-Röhre von ca. 25 nm aufbauen.
In biologischen Organismen übernehmen MT wichtige Funktionen im Bereich der Zellgestalt und der Zellbewegung. MT sind in besonderem Maße an der Aufrechterhaltung der Zellpolarität beteiligt. Zusammen mit assoziierten Proteinen (MaPs) sind MT in einer stabilen und dauerhaften Anordnung für den Gleitmechanismus in Cilien verantwortlich, einer biologischen Struktur, die darauf spezialisiert ist, wiederholte Bewegungen hervorzubringen.

Polymerisation (Aufbau) und Depolymerisation (Abbau) beruhen auf einem Austausch von Tubulin-Molekülen zwischen den MT und einem im Cytoplasma gelösten Vorrat. Innerhalb einer Zelle kann das empfindliche Gleichgewicht von Auf- und Abbau durch Substanzen gestört werden, welche die Polymerisation erhöhen oder hemmen. Dieser Vorgang kann durch Tubulin-bindende Substanzen, die sogenannten MT-Inhibitoren, blockiert werden. MT-Inhibitoren sind eine Gruppe strukturell verschiedener Verbindungen, die durch Pilze, Pflanzen, marine Organismen oder synthetisch im Labor hergestellt werden können. Colchicin, ein aus der Herbstzeitlosen gewonnenes Alkaloid bindet fest an Tubulin und verhindert dadurch eine Polymerisation. Weitere lang bekannte Hemmstoffe sind Vinblastin, Vincristin und Taxol. Taxol stabilisiert die MT-Strukturen und verhindert deren Abbau (Wilson L (1975) Life Sci. 17: 303-310). Benzimidazole (BZ) sind ein Beispiel für eine Verbindungsklasse mit einer therapeutischen Wirksamkeit gegen Helminthen (Horton RJ (1990) Parasitology Today 6(4): 106; Townsend LB and Wise DS (1990) Parasitology Today 6(4): 107-112). BZ sind synthetische Inhibitoren von Säuger-Tubulin Polymerisation in vitro, die eine höhere Affinität für das Helminthen Tubulin als für die Säugerzelle in vivo zeigen (Lacey E (1988) Int. J. Parasitol. 18, 885-936). Kompetitive Liganden bindende Studien mit H3-Colchicin bestätigen, daß BZ mit der Colchicin-bindenden Domäne interagieren und dadurch die Polymerisation verhindern (Friedman PA and Platzer EG (1978) Biochim. Acta 544, 605-614).

In Pflanzenzellen ist das MT-System vor allem an der Ausbildung von Zellform und Zellpolarität beteiligt und steuert daneben auch die Verteilung der Chromosomen in der Mitose. Herbizide, die auf das MT-System wirken, unterbrechen daher den Zellzyklus (Hess FD (1987) Rev. Weed Sci 3: 183-203). Als morphologische Folge der Herbizid-Wirkung entstehen dann Riesenzellen mit Riesenkernen, mehrkernige Zellen, Gewebeschwellungen bis hin zum endgültigen Wachstumsstop.

Obwohl die Sequenz des Tubulins vieler Arten einen hohen Grad an Homologie zeigt, bestehen zum Teil drastische Unterschiede zwischen pflanzlichem und tierischem Tubulin. So polymerisiert z.B. tierisches Tubulin in-vitro spontan, während dies für pflanzliches Tubilin nicht der Fall ist. Das heißt es ist nicht von vornherein möglich, Erkenntnisse die mit und am pflanzlichen Tubulin gewonnen wurden auf tierisches Tubulin zu übertragen und umgekehrt.

Erfindungsgemäß verwendbare Wirkstoffe sind Verbindungen die das Mikrotubulinsystem von Pflanzen hemmen. Dazu zählen bevorzugt herbizide Verbindungen der Verbindungsklassen der 2,6-Dinitroaniline und der N-Arylcarbamidsäureester.

Besonders bevorzugt sind dabei 2,6-Dinitroaniline der folgenden Formel (I) 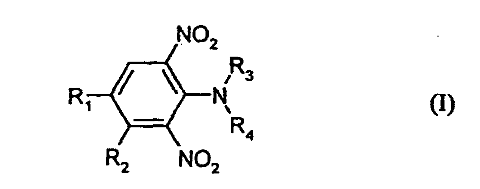 in welcher
- R₁: für Wasserstoff, Halogen, Nitro, Cyano, Alkyl, Alkenyl, Alkoxy, Alkenyloxy, Alkylthio, Alkylsulfinyl, Alkylsulfonyl, Halogenalkyl, Halogenalkenyl, Halogenalkoxy, Halogenalkenyloxy, Aminosulfonyl, Alkylsulfonylamino steht,
- R₂: für Wasserstoff, Halogen, Amino, Alkyl, Alkoxy steht,
- R₃: für Wasserstoff, Alkyl, Alkenyl, Alkinyl, Cycloalkyl-alkyl, Alkyloxy, Halogenalkyl steht,
- R₄: für Alkyl, Alkenyl, Alkinyl, Cycloalkyl-alkyl, Alkyloxy, Halogenalkyl steht,
- R₃ und R₄: gemeinsam mit dem Stickstoff an das sie gebunden sind, einen heterocyclischen Rest bilden.

Besonders bevorzugt sind die N-Arylcarbamate der Formel (II) 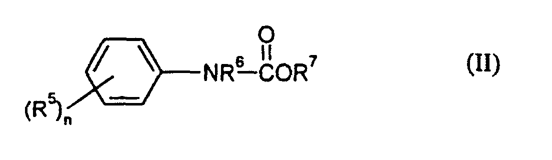 in welcher
- R⁵: für Wasserstoff, Halogen steht,
- R⁶: für Wasserstoff, Alkyl, Alkenyl steht,
- R⁷: für Alkyl, Alkenyl, Alkinyl, Halogenalkyl, Halogenalkenyl, Halogenalkinyl steht,
- n: für 1 oder 2 steht.

Gegebenenfalls substituiertes Alkyl allein oder als Bestandteil eines der Reste Alkoxy, Alkylthio, Alkylsulfinyl, Alkylsulfonyl, Halogenalkyl, Cycloalkylalkyl in den allgemeinen Formeln bedeutet geradkettiges oder verzweigtes Alkyl mit vorzugsweise 1 bis 6, insbesondere 1 bis 4 Kohlenstoffatomen. Beispielhaft seien gegebenenfalls substituiertes Methyl, Ethyl, n-Propyl, Isopropyl, n-Butyl, Isobutyl, sec-Butyl, tert-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl und 2-Ethylbutyl genannt. Vorzugsweise seien Methyl, Ethyl, n-Propyl, Isopropyl, n-Butyl, Isobutyl, sec-Butyl und tert-Butyl genannt.

Gegebenenfalls substituiertes Alkenyl allein oder als Bestandteil eines der Reste Alkenoxy, Halogenalkenyl, Halogenalkenyloxy in den allgemeinen Formeln bedeutet geradkettiges oder verzweigtes Alkenyl mit vorzugsweise 1 bis 6, insbesondere 1 bis 4 Kohlenstoffatomen. Beispielhaft seien gegebenenfalls substituiertes Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-pro-penyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-bute-nyl, 1-Ethyl-3-butenyl, 2-Ethyl-2-butenyl, 2-Ethyl-3-butenyl, 1,1,2-Trimethyl-2-prope-nyl, 1-Ethyl-1-methyl-2-propenyl und 1-Ethyl-2-methyl-2-propenyl genannt. Vorzugsweise seien gegebenenfalls substituiertes Ethenyl, 2-Propenyl, 2-Butenyl oder 1-Methyl-2-propenyl genannt.

Gegebenenfalls substituiertes Cycloalkyl allein oder als Bestandteil eines der Reste Cycloalkylalkyl in den allgemeinen Formeln bedeutet mono-, bi- und tricyclisches Cycloalkyl, vorzugsweise mit 3 bis 10, insbesondere mit 3, 5 oder 7 Kohlenstoffatomen. Beispielhaft seien gegebenenfalls substituiertes Cyclopropyl, Cyclobutyl, Cyclopentyl, Cyclohexyl, Cycloheptyl, Cyclooktyl, Bicyclo[2.2.1]heptyl, Bicyclo[2.2.2]oktyl und Adamantyl genannt.

Gegebenenfalls substituiertes Alkinyl sowie der Alkinylanteil von Halogenalkinyl in den allgemeinen Formeln bedeuten geradkettiges oder verzweigtes Alkinyl mit vorzugsweise 2 bis 6, insbesondere 2 bis 4 Kohlenstoffatomen. Beispielhaft und vorzugsweise seien gegebenenfalls substituiertes Ethinyl, Propinyl-(1), Propinyl-(2) und Butinyl-(3) genannt.

Als Halogensubstituenten der Reste Halogenalkyl, Halogenalkenyl, Halogenalkinyl, Halogenalkoxy, Halogenalkenyloxy, seien bevorzugt genannt Fluor, Chlor, Brom, insbesondere Fluor oder Brom.

Im einzelnen seien die folgenden halogensubstituierten Reste genannt Trifluormethyl, Chlordifluormethyl, Difluormethyl, Trichlormethyl, Pentafluorethyl, Chlor-fluor-ethyl, Trifluormethoxy, Perfluorethylen, Perfluorethylenoxy, Chlorpropinyl, Fluorpropinyl.

Die oben aufgeführten 2,4-Dinitroaniline und N-Arylcarbamidsäureester sind bekannte Verbindungen bzw. lassen sich einfach nach bekannten Methoden herstellen.

Zu den 2,4-Dinitroanilinen zählen die folgenden Wirkstoffe mit den common names: Trifluralin, Benfluralin, Profluralin, Dinitramine, Nitralin, Oryzalin, Isopropalin, Ethalfluralin, Dipropalin sowie Derivate und Homologe dieser Verbindungen.

Zu den N-Arylcarbamidsäureestern zählen die folgenden Wirkstoffe mit den common names: Propham (IPC), Chlorpropham (CIPC), Barban, Chlorbufam, Swep sowie Derivate und Homologe dieser Verbindungen.

Die Wirkstoffe eignen sich bei günstiger Warmblütertoxizität zur Bekämpfung von Parasiten der Gattung Neospora, die in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten.

Zu Labor- und Versuchstieren gehören Mäuse, Ratten, Meerschweinchen, Goldharnster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspensionen zur oralen oder dermalen Anwendung sowie zu Injektion; Halbfeste Zubereitungen;

Formulierungen bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramusculär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zu Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht oder durch Tauchen (Dippen, Baden oder Waschen aufgebracht. Diese Lösungen werden wie oben bei den Injektionslösungen beschrieben hergestellt.

Es kann vorteilhaft sein, bei der Herstellung Verdickungsmittel zuzufügen.
Verdickungsmittel sind: Anorganische Verdickungsmittel wie Bentonite, kolloidale Kieselsäure, Aluminiummonostearat, organische Verdickugsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt, indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß Formulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgieß Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördemde Stoffe, Antioxidantien, Lichtschutzmittel, Haftmittel zugefügt.

Als Lösungsmittel seien genannt: Wasser, Alkanole, Glycole, Polyethylenglykole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglycolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördemde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfat, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen können oral, dermal oder als Injektion angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase gelöst und diese unter Zuhilfenahme geeigneter Emulgatoren und gegebenenfalls weiterer Hilfsstoffe wie Farbstoffe, resorptionsfördemde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäurebiglycerid, Triglyceridgeanisch mit Pnanzenfettsäure der Kettenlänge C₈-C₁₂ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylenglykol-pelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/-Caprinsäureester von gesättigten Fettalkoholen der Kettenlänge C₁₂-C₁₈, Isopropylstearat, Ölsäuc-eoIeylescer, Ölsäuredecylester, Ethyloleat, Mclchsäureethylester, wachsartige Fettsäureester wie künstliches Entenhüneldrusenfett, Dibutylphthalat, Adipinsäuredüsopropylester, letzterem verwandte Estergemische u.a..

Fettalkohole wie Isotridecytaikohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglykol, Glycerin, Sorbitol und ihre Gemische.

Als Emulgatoren seien genannt:
nichtionogene Tenside, z.B. polyoxyethyliertes Rizinusöl, polyoxyethyliertes Sorbitanmonooleat, Sorbitanmonostearat, Glycerinmonostearat, Polyoxyethylstearat, Alkylphenolpolyglykolether,
ampholytische Tenside wie Di-Na-N-tauryl-β-iminodipropionat oder Lecithin;
anionaktive Tenside, wie Na-Laurytsulfat, Fettalkoholethersulfate, MonolDialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dernial oder als Injektion angewendet werden. Sie werden hergestellt indem man den Wirkstoff in einer Trägerflüssigkeit gegebenenfalls unter Zusatz weiterer Hilfsstoffe wie Netzmittel, Farbstoffe, resorptionsfördemde Stoffe, Konservierungsstoffe, Antioxidantien Lichtschutztnittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker-, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenyl-imidazolthiazol, Benzimidazolcarbamate, Praziquantel, Pyrantel, Febantel, Ivermectin.

Anwendungsfertige Zubereitungen enthalten den Wirkstoff in Konzentrationen von 10 ppm bis 20 Gew.-%, bevorzugt von 0,1 bis 10 Gew.-%.

Zubereitungen die vor Anwendung verdünnt werden, enthalten den Wirkstoff in Konzentrationen von 0,5 bis 90 Gew.-%, bevorzugt von 5 bis 50 Gew.-%.

Im allgemeinen hat es sich als vorteilhaft erwiesen, Mengen von etwa 1 bis 100 mg Wirkstoffje kg Körpergewicht pro Tag zur Erzielung wirksamer Ergebnisse zu verabreichen.

Die Verwendung der erfindungsgemäßen Wirkstoffe geht aus den nachfolgenden Beispielen hervor.

### Beispiel 1

### Testen von Substanzen an infizierten Neospora caninum Zellkulturen

Das Testen von Substanzen fand in 96-well-Platten (Falcon 3872) statt. Auf den Zellkultur-Platten wurde zunächst ein Zellkultur-Monolayer der Wirtszellen (Vero oder ED) angelegt. Hierfür wurden zwei 50 ml Gewebekulturflaschen (insgesamt 50 cm³ Zellkulturfläche) mit einem ausgebildeten nicht infizierten Monolayer benötigt. Der Zellrasen dieser Kultur wurde mit 5 ml Trypsin-EDTA (Gibco, 45300-019) im CO₂-Brutschrank bei 37°C abgelöst. Nach 10 min Inkubationszeit hatten sich die Zellen zum größten Teil abgelöst. Mit einer 5 ml-Pipette wurde die Zellsuspension in ein 50 m!-Zentrifugenröhrchen (Greiner, B 769331) überführt, indem ca. 1 ml angewärmtes foetales Kälberserum vorgelegt wurde. Nach 5 min Zentrifugation bei 1500 UpM (Varifuge 3.0, Heraeus) wurde der Überstand verworfen und das Zell-Pellet in 100 ml RPMI-Medium (95 % RPMI 1640, 2 % FCS, 1 % L-Glutamin, 1 % Natriumbicarbonat, 1 % Penicillin/Streptomycin) resuspendiert. Von dieser Zellsuspension wurden 150 µl pro well einer 96-well Platte einpipettiert. 100 ml Medium reichen für die Beschichtung von 6-Mikrowellplatten. Beschichtete Zellkultur-Platten wurden im Brutschrank bei 37°C unter 5 % CO₂ für 24 h kultiviert. Danach erfolgte eine Infektion mit Neospora canicum Tachyzoiten bei einer Konzentration von 48.000 Tachyzoiten pro well und einer weiteren Inkubation bei 37°C und 5 % CO₂ für 24 h. Die zu testenden Substanzen wurden in 1,5 ml Eppendorfreaktionsgefäße eingewogen, wobei die eingewogene Menge bei 0,5 bis 1,5 mg lag. Anschließend wurde pro mg Substanz 1 ml DMSO zupipettiert was einer Verdünnung von 1 x 10⁻³ g/ml entspricht. Das Medium zur Behandlung, in dem die weiteren Verdünnungsreihen erfolgten, bestand aus 87 % RPMI 1640, 10 % FCS, 1 % L-Glutamin, 1 % Natriumbicarbonat, 1 % Penicillin/Streptomycin. Beim ersten Screening wurden die Konzentrationen 10⁻⁵, 10⁻⁶ und 10⁻⁷ g/ml eingesetzt. 24 h nach der Infektion durch Neospora caninum wurden die verdünnten Präparate in einem Volumen von 150 µl/Well auf die Zellkulturplatten übertragen. In der ersten Reihe wurde unbehandeltes Medium verwendet, diese Reihe enthielt sowohl die infizierte Kontrolle als auch die nicht infizierte Kontrolle. Danach erfolgte eine Inkubation der Zellplatten für 5 Tage bei 37°C und 5 % CO₂. Die mikroskopische Auswertung erfolgte 4 Tage nach Behandlungsbeginn und 5 Tage nach der Infektion bei einer Vergrößerung von 25 x 10 in einem Umkehrmikroskop nach folgendem Bewertungsschema:

| **Bewertung** | **Optischer Eindruck** |
|---|---|
| 0 = keine Wirkung | Monolayer vollständig zerstört |
| 1 = schwache Wirkung | Monolayer teilweise zerstört, Parasitennester |
| | zu erkennen |
| 2 = volle Wirkung | Monolayer unzerstört, keine Tachyzoiten zu |
| | erkennen |
| T = cytotoxisch | Zellen abgestorben, abgekugelt |

### Beispiel 2

### In vitro Ergebnisse: Tabelle

| | |
|---|---|
| Testergebnisse: | Neospora caninum in Zellkultur |

| | Dosis in g/mL | | |
|---|---|---|---|
| Präparat | 10⁻⁵ | 10⁻⁶ | 10⁻⁷ |
| Benfluralin | 2 | 2 | 1 |
| Butralin | 2 | 2 | 0 |
| Chlorbufam | T/2 | 0 | |
| Chlorpropham | T/2 | 2 | 1 |
| Dinitramid | 2 | 2 | 2 |
| Enthalfluralin | 2 | 2 | 1 |
| Isopropalin | T/2 | T/2 | 0 |
| Nitralin | 2 | 2 | 0 |
| Oryzalin | T | 2 | 1 |
| Profluralin | 2 | 2 | 0 |
| Propham | T/2 | 2 | 1 |
| Trifluralin | T/2 | 2 | 1 |

Die im Beispiel verwendeten Abkürzungen haben folgende Bedeutung:
CO₂ = Kohlendioxid
DMSO = Dimethylsulfoxid
ED = Dermale Zellinie des Pferdes
EDTA = Ethylendiamin-Tetraessigsäure
FCS = Foetales Kälberserum
RPMI = Wachstumsmedium für Zellkulturen
UpM = Umdrehung pro Minute
VERO = Nierenzellinie der afrikanischen grünen Meerkatze

## Patentansprüche

1. Verwendung von Wirkstoffen, die das Mikrotubulinsystem von Pflanzen hemmen, zur Herstellung von Arzneimittel zur Bekämpfung von durch Protozoen der Gattung Neospora hervorgerufenen Erkrankungen.

2. Verwendung gemäß Anspruch 1, wobei die Protozoen der Gattung Neospora Neospora suis sind.

3. Verwendung gemäß einem der vorstehenden Ansprüche, wobei der Wirkstoff, der das Mikrotubulinsystem von Pflanzen hemmt, eine herbizide Verbindung der Verbindungsklassen der 2,6-Dinitroaniline oder der N-Arylcarbamidsäureester ist.

## Claims

1. Use of active compounds which inhibit the microtubulin system of plants for the preparation of medicaments for controlling diseases caused by protozoans of the genus Neospora.

2. Use according to Claim 1, where the protozoans belong to the genus Neospora suis.

3. Use according to one of the preceding claims, wherein the active compound which inhibits the microtubulin system of plants is a herbicidal compound from the classes of the 2,6-dinitroaniline and the N-arylcarbamate compounds.

## Revendications

1. Utilisation de substances actives qui inhibent le système de microtubuline de plantes, pour la préparation de médicaments destinés à combattre des maladies causées par des protozoaires du genre Néospora.

2. Utilisation suivant la revendication 1, dans laquelle les protozoaires du genre Néospora appartiennent à l'espèce Néospora suis.

3. Utilisation suivant l'une des revendications précédentes, dans laquelle la substance active qui inhibe le système de microtubuline de plantes est un composé herbicide des classes de composés comprenant les 2,6-dinitroaniline ou les esters d'acides N-arylcarbamiques.
